# EUROPEAN PATENT APPLICATION

(11) **EP 4 692 771 A1**
(43) Date of publication of application: **11.02.2026**
(21) Application number: 23932223.3
(22) Date of filing: 10.10.2023
(51) Int. Cl.: G01N 21/84, G01N 21/31, G01N 21/3563, G01N 21/47

(54) **SYSTEM FOR DISTINGUISHING MATERIAL OF OBJECT UNDER MEASUREMENT**

(30) Priority: 05.04.2023 KR 20230044941
(71) Applicant: Repla Inc., Gyeongsan-si, Gyeongsangbuk-do 38541 (KR)
(72) Inventor: SUH, Dong Eun, Yongin-si Gyeonggi-do 17103 (KR); JEON, Byoung Jin, Yongin-si Gyeonggi-do 17084 (KR); RYU, Kyoung Ho, Gwangju-si Gyeonggi-do 12772 (KR)
(74) Representative: Elzaburu S.L.P.
(86) International application number: PCT/KR2023/015547
(87) International publication number: WO 2024/210289

(57) **Abstract**

The system for distinguishing the material of an object under measurement, according to the present invention, comprises: a lighting device which comprises a light-emitting unit for emitting light toward an object under measurement; and a scanner which comprises an NIR sensor, and which is provided to distinguish the material of the object under measurement on the basis of pre-input information and light that is generated by each of the NIR sensor and the light-emitting unit, penetrates the object under measurement, and is then received.

## Description

### [Technical Field]

The present invention relates to a system for distinguishing a material of an object under measurement, and more particularly, to a system for distinguishing a material of an object under measurement, which is capable of generating light from below the object under measurement toward the object under measurement and diffusing the generated light to increase the amount of light received by a scanner.

### [Background Art]

Representative examples of plastics used as recycling agents include PET, PE, PP, ABS, and PVC.

In recent years, importance of separate collection of recycled products has emerged due to environmental problems.

Accordingly, many plastic scanners have recently been developed which may be easily carried by a user and used to distinguish plastic materials regardless of a location or time.

Near infrared ray (NIR) sensors are applied to plastic scanners that have been developed recently.

Generally, the NIR sensors have a near-infrared wavelength of 750 nm to 2,500 nm, particularly, a wavelength close to red visible light. Accordingly, the NIR sensors are widely used in various fields such as infrared cameras, infrared communication, remote controls for home appliances, and biometric authentication (vein authentication).

Plastic scanners to which the NIR sensors are applied are devices that generate near-infrared rays toward an object under measurement and receive light reflected from the object under measurement to distinguish a material of the object under measurement.

However, when the material of the object under measurement formed of a transparent material is distinguished using the plastic scanner to which the NIR sensor according to the related art is applied, the amount of light received by the plastic scanner after the light generated from the plastic scanner passes through the object under measurement and is reflected from the object under measurement is very small. When the amount of light received by the plastic scanner decreases, an operation of distinguishing the plastic material by the plastic scanner is not performed normally, and thus it is difficult to accurately distinguish the material of the object under measurement.

### [Detailed Description of Invention]

### [Technical Problem]

The purpose of the present invention is to provide a system for distinguishing a material of an object under measurement, which is capable of generating light from below the object under measurement toward the object under measurement and diffusing the generated light to increase the amount of light received by a scanner.

Further, the purpose of the present invention is to provide a system for distinguishing a material of an object under measurement, which achieves easy portability and mobility.

The problems to be solved by the present invention are not limited to the problems described above, and those skilled in the art will clearly understand other problems not described based on the following description.

### [Technical Solution]

According to the present invention, the purpose may be achieved by a system for distinguishing a material of an object under measurement, which includes a lighting device including a light emitting unit that emits light toward an object under measurement and a scanner that includes a near infrared ray (NIR) sensor and distinguishes a material of the object under measurement based on light generated by the NIR sensor and the light emitting unit and received after passing through the object under measurement and previously input information.

The input information to the scanner may be a wavelength band of light corresponding to the material of the object under measurement to be distinguished.

The scanner may be provided to distinguish a plastic material of the object under measurement and receive information on a wavelength band of light corresponding to at least one type of plastic material corresponding to the plastic material to be distinguished.

The lighting device may be disposed to face the scanner, and the object under measurement may be positioned between the lighting device and the scanner.

The lighting device may have a seating portion on which the object under measurement is seated.

The light emitting unit may include a halogen lamp.

In the lighting device, the light emitting unit may be spaced a predetermined distance from the NIR sensor and disposed in a circumferential direction of the NIR sensor.

The lighting device may further include a diffusion member that diffuses the light generated by the light emitting unit before the light reaches the object under measurement.

The lighting device may include a controller that is connected to the light emitting unit and controls the light emitting unit to be turned ON/OFF, a battery that supplies electric power to the light emitting unit, and a switch that is connected to the controller, is connected to the battery, and operates the light emitting unit.

The lighting device may further include a casing on which the object under measurement is seated, which has an internal space in which the battery and the controller are mounted, and which has an opening hole of which at least a partial area is open so that the light generated by the light emitting unit faces the object under measurement, and the diffusion member may be mounted on the opening hole of the casing.

The controller may include a charging identification member that is connected to the battery and displays a charging state of the battery, and the charging identification member may include a light emitting diode (LED) lamp that is mounted between the light emitting units and identifies the charging state of the battery through the opening hole.

The battery may be provided to be charged/discharged, and a charging terminal that is connected to the battery and supplies electric power to the battery may be provided in the controller.

The switch and the charging terminal may be exposed at a side surface of the casing.

The system may further include a battery housing in which the battery is accommodated, and the battery housing may be mounted inside the casing.

The casing may include at least one gripping protrusion on a side surface thereof.

The casing may include at least one slip preventing member on a bottom surface thereof.

The casing may include an upper casing in which the opening hole is formed and at least one coupling protrusion is provided and a lower casing in which the battery and the controller are mounted and at least one coupling groove to which the coupling protrusion is coupled is provided, a support rib that supports the battery accommodated in the lower casing may protrude from an inner surface of the upper casing, and the upper casing and the lower casing may be separated from each other by the coupling protrusion and the coupling groove.

### [Advantageous Effects]

According to a system for distinguishing a material of an object under measurement according to an embodiment of the present invention, light can be generated and diffused from below the object under measurement toward the object under measurement and a scanner using a lighting device, and thus the amount of light received by the scanner after passing through the object under measurement can increase. As the amount of light received by the scanner increases, reliability and accuracy for distinguishing the material of the object under measurement using the scanner can be improved.

Further, according to a system for distinguishing a material of an object under measurement according to the embodiment of the present invention, a lighting device is mounted inside a casing, and thus portability and mobility of the system for distinguishing a material of an object under measurement can be improved. Accordingly, it is possible to distinguish a material of an object under measurement to be distinguished as needed regardless of a location or time.

The effects of the present invention are not limited to the effects described above, and other effects that are not described will be clearly understood by those skilled in the art to which the present invention pertains based on the following description.

### [Description of Drawings]

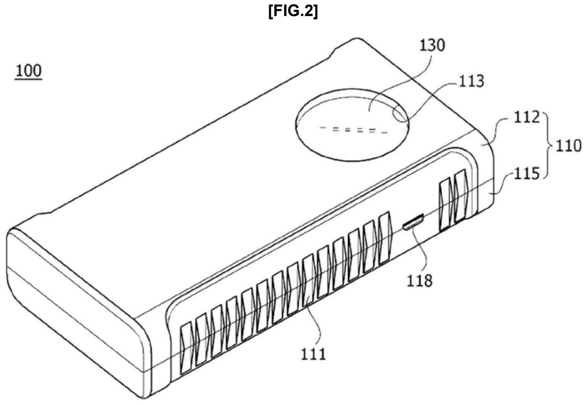

FIG. 2 is a perspective view of a lighting device illustrated in FIG. 1.

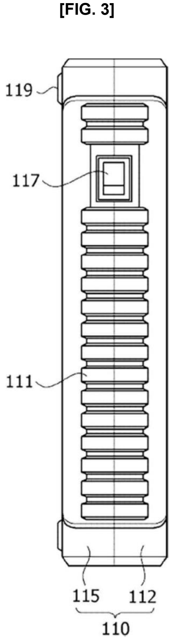

FIG. 3 is a left perspective view of the lighting device illustrated in FIG. 2.

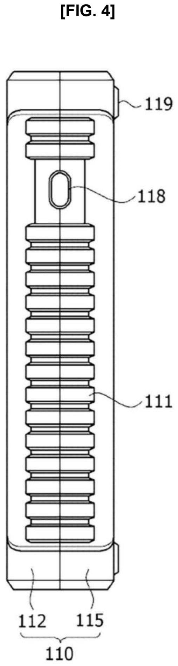

FIG. 4 is a right perspective view of the lighting device illustrated in FIG. 2.

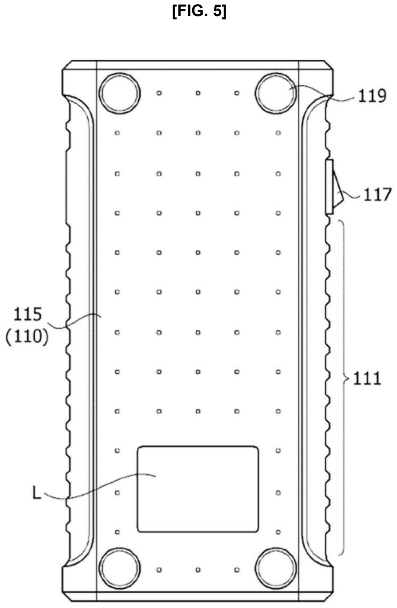

FIG. 5 is a rear view of the lighting device illustrated in FIG. 2.

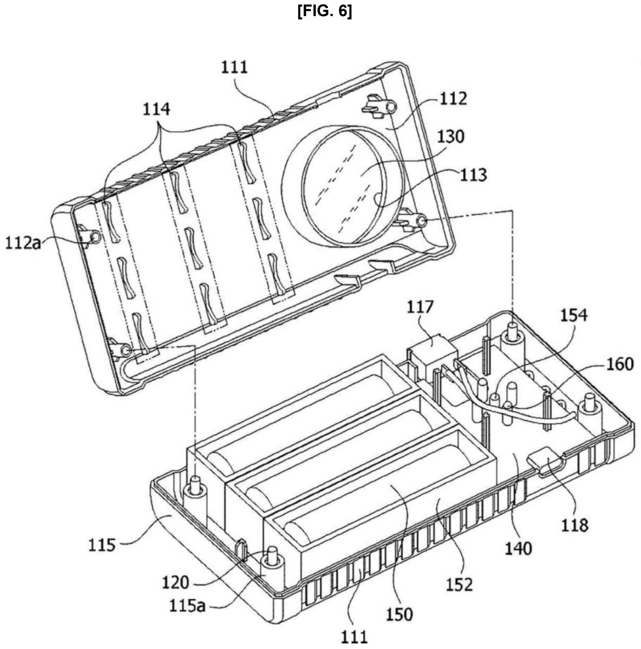

FIG. 6 is a view illustrating an internal structure of the lighting device illustrated in FIG. 2.

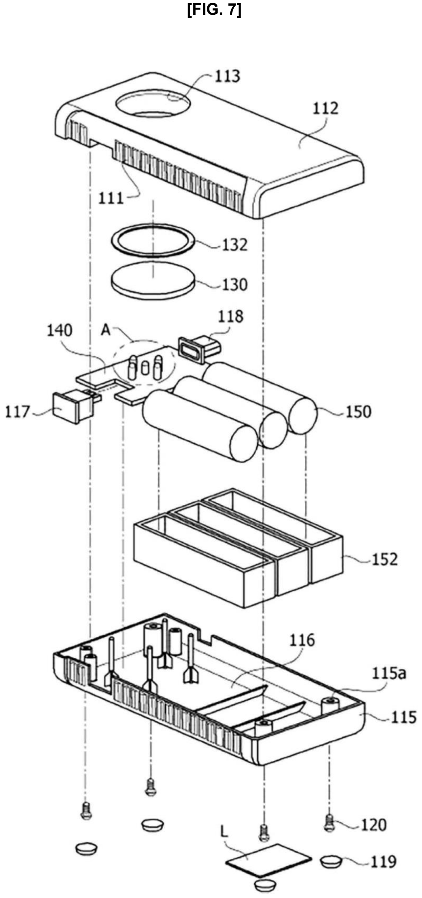

FIG. 7 is an exploded perspective view of the lighting device illustrated in FIG. 2.

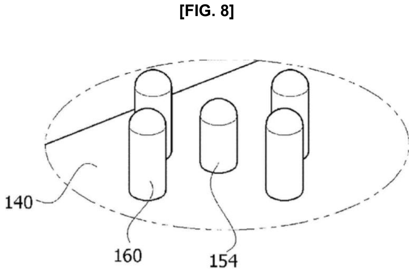

FIG. 8 is an enlarged view of part A illustrated in FIG. 7.
FIG. 9 is a view illustrating an arrangement of a light emitting unit illustrated in FIG. 8.
FIG. 10 is a perspective view of a scanner illustrated in FIG. 1.
FIG. 11 is a schematic view illustrating a method of distinguishing a material of an object under measurement using a system for distinguishing a material of an object under measurement illustrated in FIG. 1.
FIG. 12 is a view for describing a diameter of the light emitting unit and a diameter of a diffusion member for a near infrared ray (NIR) sensor mounted on a sensing unit of the scanner illustrated in FIG. 11.
FIG. 13 is a graph showing a result of distinguishing an object under measurement made of a transparent PET material using the system for distinguishing a material of an object under measurement illustrated in FIG. 1.
FIG. 14 is a graph showing a result of distinguishing the object under measurement made of a transparent PET material using a conventional plastic scanner.
FIG. 15 is a graph showing a result of distinguishing an object under measurement made of an opaque PET material using the conventional plastic scanner.
FIG. 16 is a graph showing a result of distinguishing an object under measurement made of a transparent PS material using the system for distinguishing a material of an object under measurement illustrated in FIG. 1.
FIG. 17 is a graph showing a result of distinguishing the object under measurement made of a transparent PS material using the conventional plastic scanner.
FIG. 18 is a graph showing a result of distinguishing an object under measurement made of an opaque PS material using the conventional plastic scanner.
FIG. 19 is a graph showing a result of distinguishing an object under measurement made of a transparent PC material using the system for distinguishing a material of an object under measurement illustrated in FIG. 1.
FIG. 20 is a graph showing a result of distinguishing the object under measurement made of a transparent PC material using the conventional plastic scanner.
FIG. 21 is a graph showing a result of distinguishing an object under measurement made of an opaque PC material using the conventional plastic scanner.

### [Modes of the Invention]

Hereinafter, a system 1000 for distinguishing a material of an object under measurement according to an embodiment of the present invention will be described in detail with reference to the accompanying drawings.

Further, the same or similar reference numbers are assigned to the same or corresponding components throughout the drawings, duplicated descriptions thereof will be omitted, and for convenience of description, a size and shape of each component illustrated may be exaggerated or reduced.

The system 1000 for distinguishing a material of an object under measurement according to the embodiment of the present invention is a system for distinguishing a material of an object 10 under measurement while in contact with the object 10 under measurement to be distinguished.

In particular, the system 1000 for distinguishing a material of an object under measurement according to the embodiment of the present invention may distinguish the material of the object 10 under measurement having polyethylene terephthalate (PET), a polyester clothing material, polyvinyl chloride (PVC), polystyrene (PS), polypropylene (PP), polyethylene (PE), acrylonitrile, butadiene, and styrene (ABS), polylactic acid (PLA), and polycarbonate (PC).

Preferably, the system 1000 for distinguishing a material of an object under measurement may distinguish which material among PP, PE, PS, PC, PVC, PET, and ABS, of which the object 10 under measurement is made.

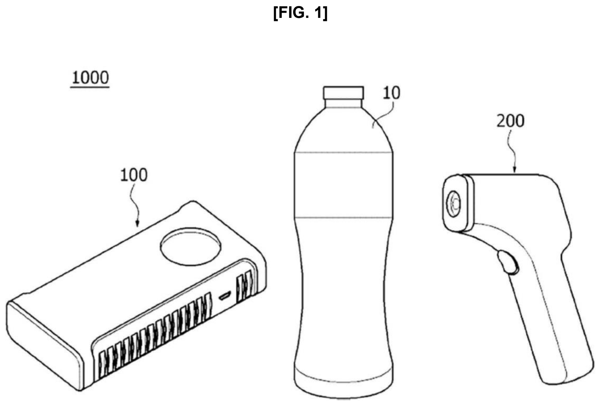

Referring to FIG. 1, the system 1000 for distinguishing a material of an object under measurement according to the embodiment of the present invention may include a lighting device 100 including a light emitting unit 160 that emits light toward the object 10 under measurement and a scanner 200 including a near infrared ray (NIR) sensor 221 and provided to distinguish the material of the object 10 under measurement based on light generated by the NIR sensor 221 and the light emitting unit 160 and received after passing through the object 10 under measurement and previously input information.

Here, a wavelength band of light corresponding to the material of the object 10 under measurement to be distinguished may be input to the scanner 200 in advance.

In other words, the scanner 200 may be provided to distinguish a plastic material of the object 10 under measurement, and in particular, information on a wavelength band of light corresponding to at least one type of plastic material corresponding to a plastic material to be distinguished may be input.

A method of distinguishing the material of the object 10 under measurement using the scanner 200 will be described in detail below.

The lighting device 100 may be disposed to face the scanner 200.

In this case, the object 10 under measurement may be positioned between the lighting device 100 and the scanner 200.

In particular, the light emitting unit 160 of the lighting device 100 generates light while facing the object 10 under measurement and disposed below the object 10 under measurement, and the light generated by the lighting device 10 passes through the object 10 under measurement and is received by the scanner 200 together with light (NIR) radiated from the scanner 200.

In this way, as the light emitting unit 160 is disposed under the object 10 under measurement and positioned symmetrically with the scanner 200, the light generated by the light emitting unit 160 may be better received by the NIR sensor 221 of the scanner 200.

The light emitting unit 160 may be provided in at least one of a light emitting diode (LED) lamp and a halogen lamp. It is preferable that the light emitting unit 160 according to the embodiment of the present invention be provided in a halogen lamp. Accordingly, the light emitting unit 160 may generate light having a wavelength band corresponding to an acceptable light source for the NIR sensor 221 mounted on the scanner 200.

Further, the light emitting unit 160 may include a plurality of halogen lamps.

For example, the light emitting unit 160 may include one to six lamps, and preferably, may include four lamps.

Further, the light emitting unit 160 may be spaced apart from the NIR sensor 221 by a predetermined distance and may be disposed in a circumferential direction around the NIT sensor.

Meanwhile, the lighting device 100 may further include a diffusion member 130.

The diffusion member 130 may diffuse light generated before the light generated by the light emitting unit 160 reaches the object 10 under measurement.

The diffusion member 130 may cover the light emitting unit 160 so that the light generated by the light emitting unit 160 is diffused toward the scanner 200.

Further, the diffusion member 130 may be formed of a transparent material.

The diffusion member 130 may be made of a film, glass, or a plastic material having a predetermined thickness, but the present invention is not necessarily limited thereto.

In this way, as the light generated by the light emitting unit 160 is diffused by the diffusion member 130, the amount of light received by the scanner 200 may increase, and thus a material distinguishment error of the object 10 under measurement using the scanner 200 may be improved.

Further, the lighting device 100 may include a controller 140, a battery 150, and a switch 117.

The controller 140 may be connected to the light emitting unit 160 and may be provided to control the light emitting unit 160 to be turned ON/OFF.

The controller 140 may be provided in a printed circuit board (PCB) form, but the present invention is not limited thereto.

The battery 150 may supply electric power to the light emitting unit 160.

The battery 150 may be provided to enable repetitive charging/discharging. For example, the battery 150 may be provided as a lithium-ion battery.

Further, the battery 150 may be connected to the light emitting unit 160 while accommodated in a battery housing 152.

Meanwhile, the battery 150 may be connected to the controller 140.

Accordingly, the controller 140 may be provided with a charging terminal 118 connected to the battery 150. For example, the charging terminal 118 may be of a C-type.

The switch 117 may operate the light emitting unit 160.

The switch 117 may be connected to the controller 140, may be connected to the battery 150, and may operate the light emitting unit 160.

For example, the switch 117 may be provided so that the light emitting unit 160 is selectively turned ON/OFF to determine an operation state of the light emitting unit 160.

To this end, the switch 117 may be provided in the form of a button so that a user may conveniently perform an operation.

Further, the lighting device 100 may include a casing 110.

The casing 110 may have an internal space 116 in which the object 10 under measurement is seated and in which the battery housing 152 in which the battery 150 is accommodated and the controller 140 are mounted.

Further, an opening hole 113 of which at least a partial area is open may be formed in the casing 110 so that the light generated by the light emitting unit 160 mounted on the controller 140 faces the object 10 under measurement.

In this case, the diffusion member 130 may be mounted on the opening hole 113.

Here, the opening hole 113 and the diffusion member 130 are illustrated in a circular shape, but the present invention is not limited thereto, and the opening hole 113 and the diffusion member 130 may be formed in various shapes such as an elliptic shape and a quadrangular shape as needed.

As the diffusion member 130 is mounted on the opening hole 113 of the casing 110, the light generated by the light emitting unit 160 is diffused to pass through the object 10 under measurement, and thus the amount of light received by the scanner 200 increases.

Meanwhile, a buffer member 132 may be provided between the opening hole 113 and the diffusion member 130.

The buffer member 132 may be provided in the form of a ring to have a predetermined thickness. For example, the buffer member 132 may be formed of foam, rubber, silicon, or the like.

Here, since the diffusion member 130 is made of a material such as plastic or glass having a relatively thin thickness, there is a risk of it being damaged by an external impact or pressure. As the buffer member 132 is mounted between the casing 110 and the diffusion member 130, the possibility of damage to the diffusion member 130 may be reduced.

Further, the controller 140 may include a charging identification member 154.

The charging identification member 154 may be connected to the battery 150 and display a charging state of the battery 150 so that the user may visually identify the charging state of the battery 150.

For example, the charging identification member 154 may include an LED lamp. Accordingly, the charging identification member 154 may be set to be turned on in red while the battery 150 is charged and may be set to be turned on in green when the charging of the battery 150 is completed. Further, the LED lamp may be set not to be turned on when the battery 150 is not being charged.

Further, the charging identification member 154 may be provided between the light emitting units 160.

The user may detect a lighting state and a light emitting state of the charging identification member 154 through the diffusion member 130 mounted on the opening hole 113 of the casing 110.

Meanwhile, the light emitting units 160 may be arranged to be spaced a predetermined distance from each other along a circumference around the charging identification member 154.

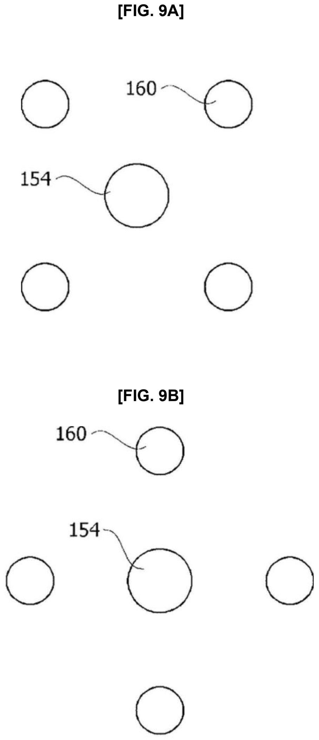

For example, as illustrated in FIGS. 9A and 9B, the light emitting units 160 may be arranged in various forms around the charging identification member 154. In this case, the arrangement of the light emitting units 160 is not limited thereto.

Further, partial areas of the switch 117 and the charging terminal 118 may be exposed on a side surface of the casing 110. Accordingly, the user may easily operate the light emitting unit 160 using the switch 117 and may easily charge the battery 150 using the charging terminal 118.

Meanwhile, the casing 110 may be provided in a detachable form.

For example, the casing 110 may include an upper casing 112 and a lower casing 115.

An outer upper end surface of the upper casing 112 may be a seating portion on which the object 10 under measurement is seated.

Further, the opening hole 113 on which the diffusion member 130 is mounted may be formed in an upper end surface of the upper casing 112.

The internal space 116 in which the battery housing 152 in which the battery 150 is accommodated and the controller 140 are mounted may be provided in the lower casing 115. The battery housing 152 and the controller 140 may be mounted in the internal space 116 by a coupling means (not illustrated) such as a bolt.

A support rib 114 may be provided on an inner surface of the upper casing 112.

The support rib 114 is a member for preventing the battery 150 mounted inside the lower casing 115 from moving.

The support rib 114 may be provided to protrude from the inner surface of the upper casing 112 toward the lower casing 115. Further, the support rib 114 may be provided such that at least a partial area thereof is formed in a curved surface to surround the battery 150.

For example, a plurality of support ribs 114 may be provided along the inner surface of the upper casing 112 at predetermined intervals. In this case, the number of the support ribs 114 may correspond to the number of the batteries 150 mounted inside the lower casing 115.

Further, at least one coupling protrusion 112a may be provided inside the upper casing 112, and at least one coupling groove 115a coupled to the coupling protrusion 112a of the upper casing 112 may be provided in the lower casing 115.

In a state in which the coupling protrusion 112a of the upper casing 112 and the coupling groove 115a of the lower casing 115 are coupled to each other, the upper casing 112 and the lower casing 115 are coupled to each other by a coupling member 120 inserted from a lower side of the lower casing 115 toward the upper casing 112.

Further, the casing 110 may be formed of a plastic material having a light weight to facilitate portability and mobility.

In this case, the casing 110 may be moved in a process of distinguishing a plastic material of the object 10 under measurement in a state in which the casing 110 is placed on the floor. In this way, in order to prevent the casing 110 from moving, the lower casing 115 may include a slip preventing member 119.

The slip preventing member 119 may be provided on a bottom surface of the lower casing 115. Further, the slip preventing member 119 may be provided at each corner of the lower casing 115.

The slip preventing member 119 may be made of silicon, rubber, or the like, but the present invention is not necessarily limited thereto.

Here, the slip preventing member 119 may prevent slipping of the casing 110, and at the same time, cover an insertion hole (not illustrated) into which the coupling member 120 coupling the upper casing 112 and the lower casing 115 is inserted.

Further, at least one label "L" may be mounted on the bottom surface of the lower casing 110. For example, the label "L" may include information on the lighting device 100.

Further, the casing 110 may include at least one gripping protrusion 111 on a side surface thereof.

In other words, the gripping protrusions 111 may be provided on opposite sides of the upper casing 112 and the lower casing 115.

The gripping protrusion 111 may allow the user to grip the casing 110 with his/her hand, and accordingly, convenient portability and mobility may be achieved.

Meanwhile, the system 1000 for distinguishing a material of an object under the measurement according to the embodiment of the present invention distinguishes the material of the object 10 under measurement to be measured through the amount of light received by the scanner 200.

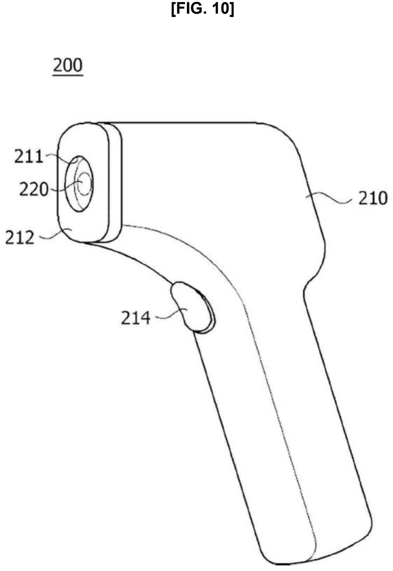

Referring to FIG. 10, the scanner 200 according to the embodiment of the present invention may include a body 210, a sensing unit 220 on which the NIR sensor 221 is mounted, a front cover 212 that covers the sensing unit 220, the operation switch 117 that is provided on the body 210 and is operated to distinguish the plastic material of the object 10 under measurement using the sensing unit 220 while in contact with the object 10 under measurement, and a result identification unit 230 indicating the distinguishment result of the plastic material of the object 10 under measurement distinguished by operating the operation switch 117.

The sensing unit 220 may be provided such that the NIR sensor 221 is mounted in a central area thereof, but the present invention is not necessarily limited thereto.

The operation switch 117 may be provided at a portion at which a thumb is positioned when the user grips the operation switch 117 with his/her hand. Accordingly, the operation switch 117 of the scanner 200 may be conveniently operated.

The result identification unit 230 may be provided as an LCD panel.

The material of the object 10 under measurement distinguished by the scanner 200 may be displayed on the result identification unit 230. Further, a graph including a wavelength band corresponding to the material of the object 10 under measurement may be displayed. Further, as needed, purity of the plastic material included in in the distinguished object 10 under measurement may be set to be displayed.

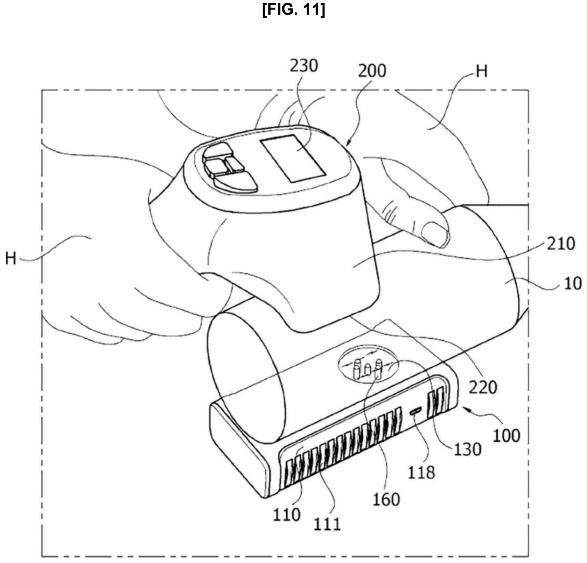

Hereinafter, an order of use of the system 1000 for distinguishing a material of an object under measurement according to the embodiment of the present invention will be briefly described with reference to FIG. 11.

First, the user generates light in the light emitting unit 160 of the lighting device 100.

Next, the user places the object 10 under measurement on the lighting device 100 and fixes the object 10 under measurement with one hand "H."

Next, the user makes contact with the object 10 under measurement using the scanner 200 with the other hand "H" and then presses the operation switch 117 of the scanner 200.

Then, the light generated and diffused from the light emitting unit 160 passes through the object 10 under measurement, and the passing light is received by the sensing unit 220 of the scanner 200.

In this case, predetermined light may be emitted toward the object 10 under measurement even in the NIR sensor 221 mounted on the sensing unit 220 of the scanner 200.

The scanner 200 may come into contact with the object 10 under measurement placed on the lighting device 100 to distinguish the material of the object 10 under measurement.

In particular, the scanner 200 receives light that is radiated by the NIR sensor 221 and passes through the object 10 under measurement and light that is generated and diffused from the lighting device 100 and passes through the object 10 under measurement.

The scanner 200 distinguishes the material of the object 10 under measurement based on the amount of received light and previously input information.

**As** described above, the information previously input to the scanner 200 may be a wavelength band of light corresponding to the material of the object 10 under measurement to be distinguished.

For example, when the amount of light received by the scanner 200 is about 1,541 DN to 2,445 DN, a peak corresponding to the wavelength (nm) corresponding to the amount (intensity) of received light is generated, and the material of the object 10 under measurement is distinguished through the wavelength in which the peak is generated.

Meanwhile, a diameter D1 of the sensing unit 220 of the scanner 200 may be equal to or smaller than a diameter D2 of the diffusion member 130 mounted on the opening hole 113 of the casing 110.

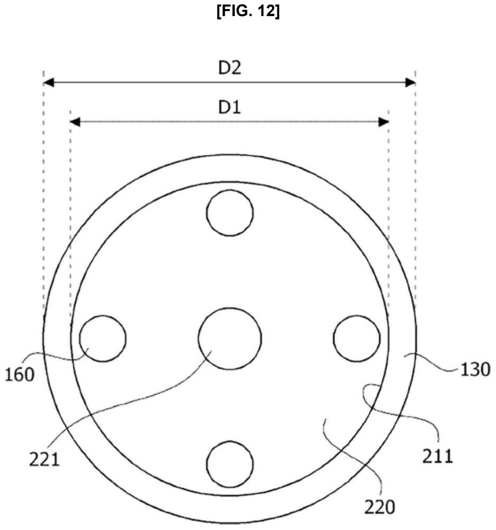

For example, referring to FIG. 12, the diameter D2 of the diffusion member 130 may be greater than the diameter D1 of the sensing unit 220 of the scanner 200.

Accordingly, the light emitting unit 160 covered by the diffusion member 130 may be positioned inside the sensing unit 220.

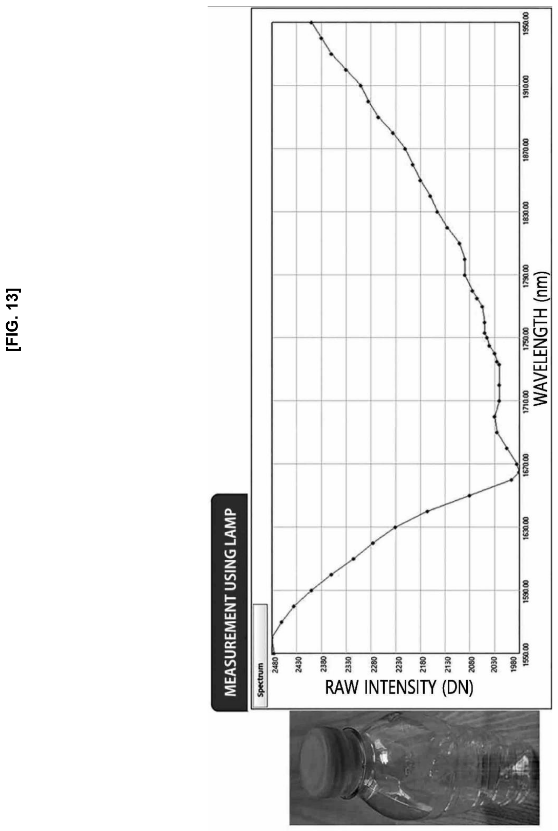

Hereinafter, a result of distinguishing the plastic material of the object 10 under measurement using the system 1000 for distinguishing a material of an object under measurement according to the embodiment of the present invention will be briefly described with reference to FIGS. 13 to 21.

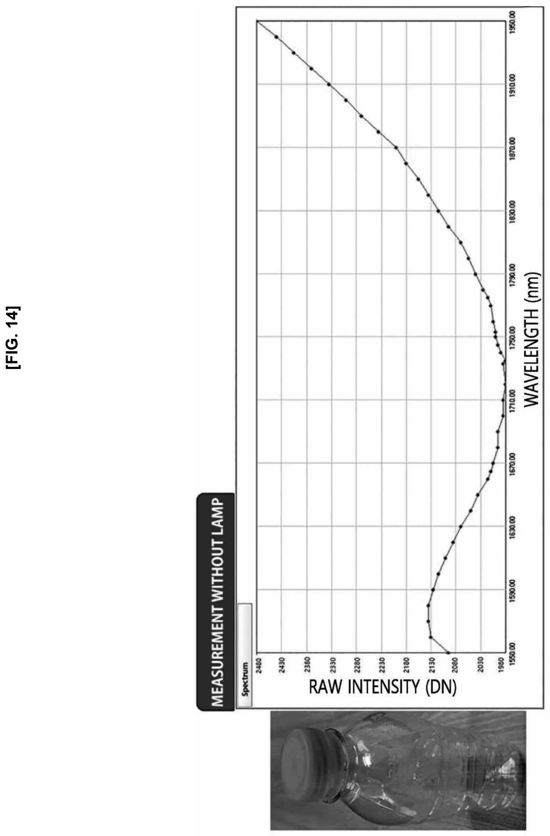

First, the object 10 under measurement made of a transparent PET material is distinguished with reference to FIGS. 13 and 14. In FIGS. 13 and 14, a left picture corresponds to the object 10 under measurement made of a transparent PET material.

As illustrated in FIG. 13, in the system 1000 for distinguishing a material of an object under measurement according to the embodiment of the present invention, light is generated and diffused from the lighting device 100 toward the object 10 under measurement made of a transparent PET material, and thus the amount (raw intensity) of light received by the scanner 200 is about 1,989 DN to 2,445 DN. Accordingly, it may be seen that the peak is generated at 1,665 nm, which is the wavelength of the object 10 under measurement made of a transparent PET material.

In contrast, as illustrated in FIG. 14, when the object 10 under measurement made of a transparent PET material is distinguished only using the scanner 200 without using the lighting device 100 as in the related art, the amount of light received by the scanner 200 (the amount of received light) is about 1,541 nm to 1,702 nm. Accordingly, it may be seen that the amount of light received by the scanner 200 is insufficient, and accordingly, the peak is not generated in the wavelength of the object 10 under measurement made of a transparent PET material.

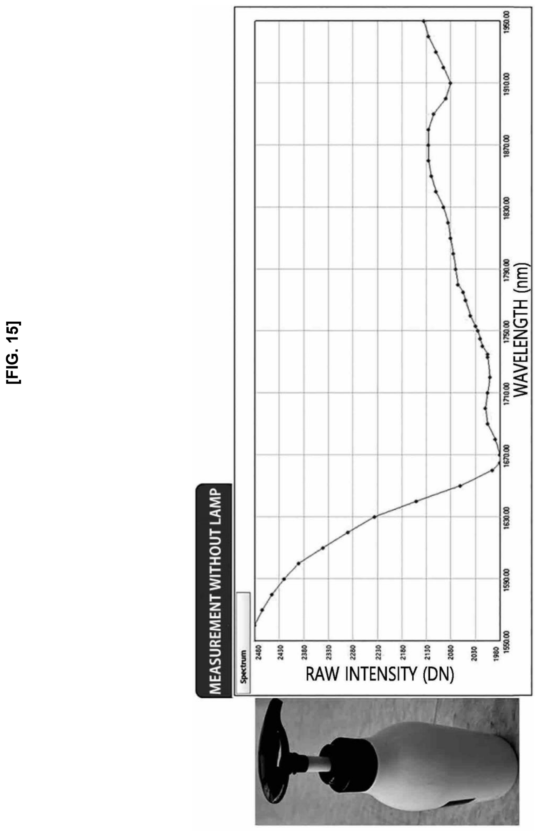

For reference, in FIG. 15, a left picture corresponds to the object 10 under measurement made of an opaque PET material.

As illustrated in FIG. 15, it may be seen that, when the object 10 under measurement made of a transparent PET material is distinguished only using the scanner 200 without using the lighting device 100, the peak is generated at 1,665 nm, which is the wavelength of the object 10 under measurement made of an opaque PET material. Here, in the case of an opaque PET material, since the light is normally reflected by the object 10 under measurement without additional light, the peak is generated at the same wavelength as in a case in which a transparent PET material is distinguished without additional light.

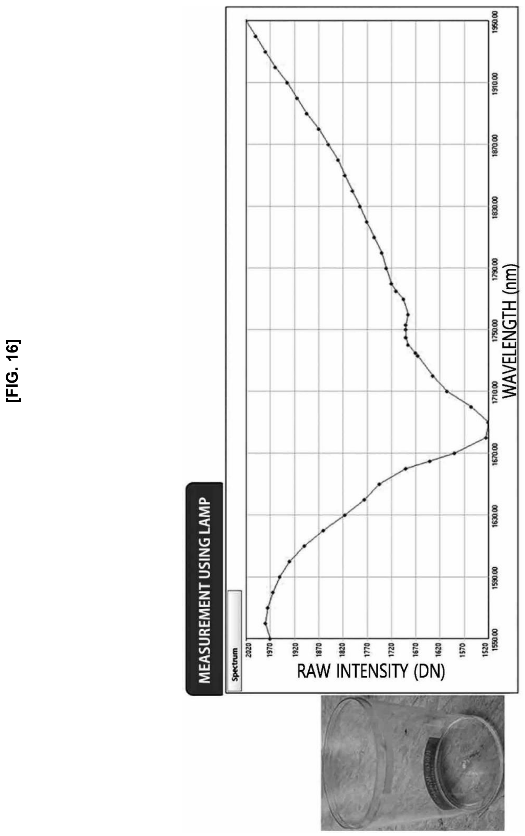

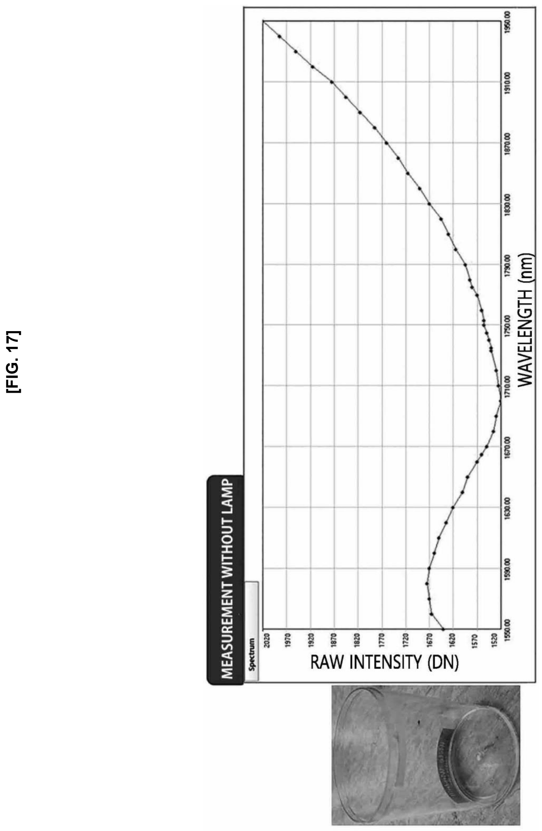

Further, the object 10 under measurement made of a transparent PS material is distinguished with reference to FIGS. 16 and 17. In FIGS. 16 and 17, a left picture corresponds to the object 10 under measurement made of a transparent PS material.

As illustrated in FIG. 16, in the system 1000 for distinguishing a material of an object under measurement according to the embodiment of the present invention, light is generated and diffused from the lighting device 100 toward the object 10 under measurement made of a transparent PS material, and thus the amount of light received by the scanner 200 is about 1,522 DN to 1,989 DN. Accordingly, it may be seen that the peak is generated at 1,690 nm, which is the wavelength of the object 10 under measurement made of a transparent PS material.

In contrast, as illustrated in FIG. 17, when the object 10 under measurement made of a transparent PS material is distinguished only using the scanner 200 without using the lighting device 100 as in the related art, the amount of light received by the scanner 200 (the amount of received light) is about 1,500 DN to 1,699 DN. In this case, it may be seen that the amount of light received by the scanner 200 is insufficient, and thus the peak is not generated in the wavelength of the object 10 under measurement made of a transparent PS material.

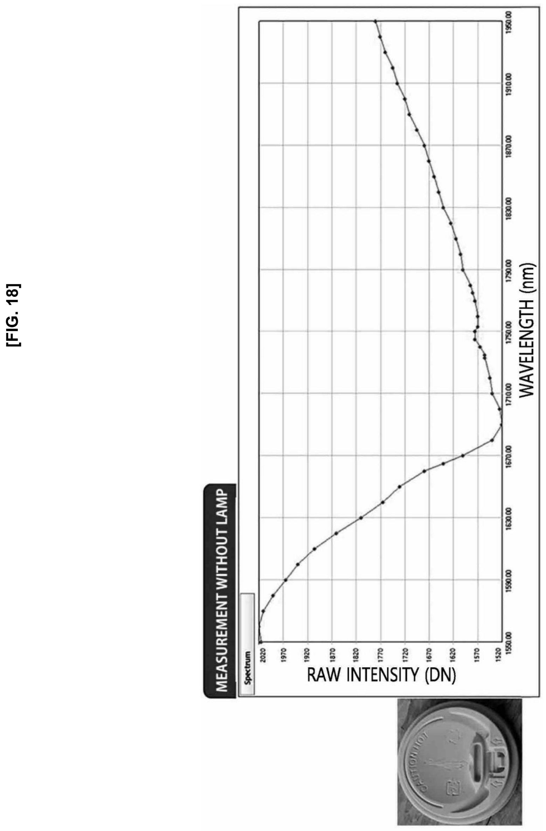

For reference, in FIG. 18, a left picture corresponds to the object 10 under measurement made of an opaque PS material.

As illustrated in FIG. 18, it may be seen that, when the object 10 under measurement made of a transparent PS material is distinguished only using the scanner 200 without using the lighting device 100, the peak is generated at 1,690 nm, which is the wavelength of the object 10 under measurement made of an opaque PS material. Here, in the case of an opaque PS material, since the light is normally reflected by the object 10 under measurement without additional light, the peak is generated at the same wavelength as in a case in which a transparent PS material is distinguished without additional light.

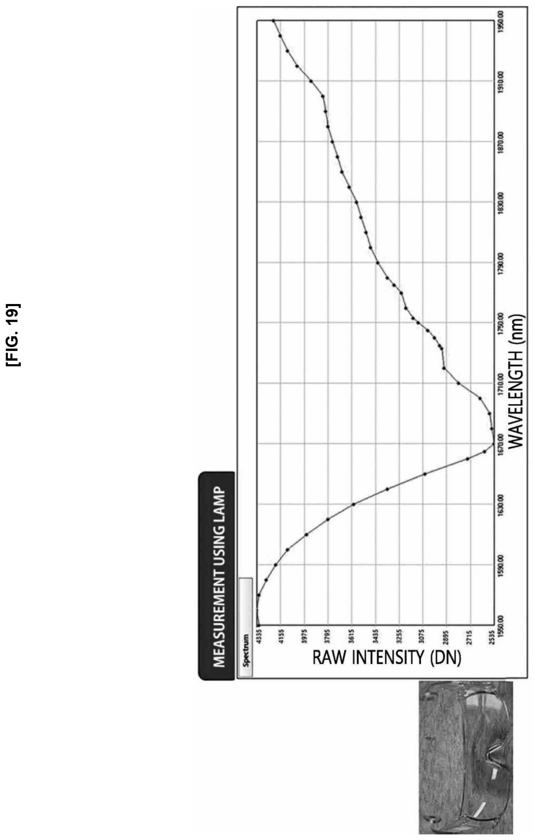

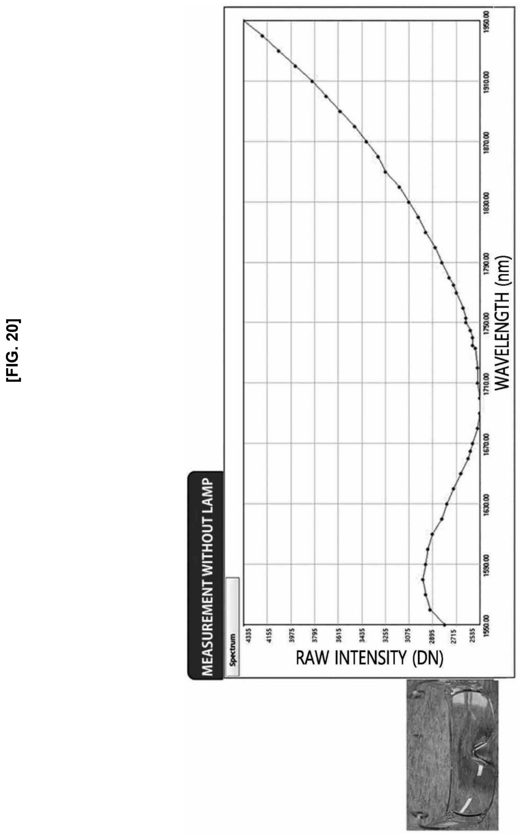

Further, the object 10 under measurement made of a transparent PC material is distinguished with reference to FIGS. 19 and 20. In FIGS. 19 and 20, a left picture corresponds to the object 10 under measurement made of a transparent PC material.

As illustrated in FIG. 19, in the system 1000 for distinguishing a material of an object under measurement according to the embodiment of the present invention, light is generated and diffused from the lighting device 100 toward the object 10 under measurement made of a transparent PC material, and thus the amount (the amount of received light) of light received by the scanner 200 is about 2,536 DN to 4,334 DN. Accordingly, it may be seen that the peak is generated at 1,690 nm, which is the wavelength of the object 10 under measurement made of a transparent PC material.

In contrast, as illustrated in FIG. 20, when the object 10 under measurement made of a transparent PC material is distinguished only using the scanner 200 without using the lighting device 100 as in the related art, the amount of light received by the scanner 200 is about 1,063 DN to 1,233 DN. Accordingly, it may be seen that, since the amount of light received by the scanner 200 is insufficient, the peak is not generated in the wavelength of the object 10 under measurement made of a transparent PC material.

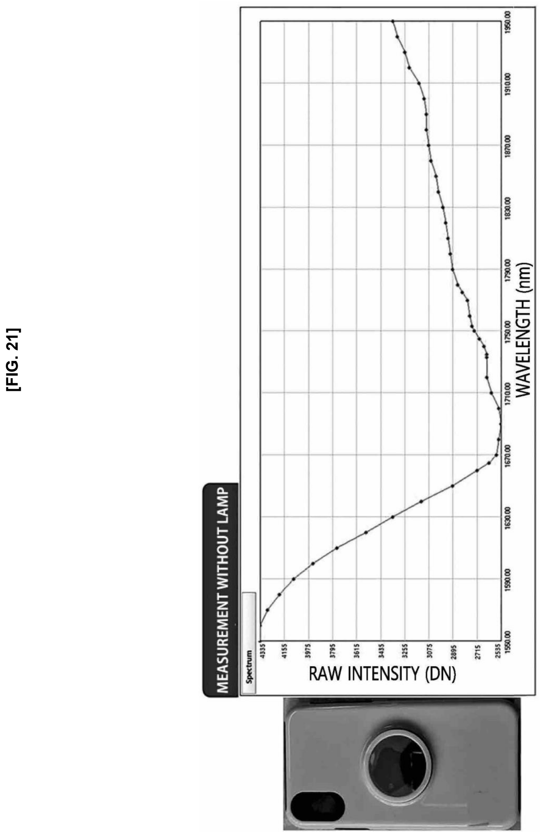

For reference, in FIG. 21, a left picture corresponds to the object 10 under measurement made of an opaque PC material.

As illustrated in FIG. 21, it may be seen that, when the object 10 under measurement made of a transparent PC material is distinguished only using the scanner 200 without using the lighting device 100, the peak is generated at 1,690 nm, which is the wavelength of the object 10 under measurement made of an opaque PC material. In the case of an opaque PC material, since the light is normally reflected by the object 10 under measurement without additional light, the peak is generated at the same wavelength as in a case in which a transparent PC material is distinguished without additional light.

Exemplary embodiments of the present invention as described above are merely disclosed for illustrative purposes, those skilled in the art having general knowledge of the present invention may make various modifications, changes, and additions within the spirit and scope of the present invention, and these modifications, changes, and additions belong to the appended claims.

### [Industrial Applicability]

According to a system for distinguishing a material of an object under measurement related to an embodiment of the present invention, the amount of light received by a scanner may be increased by a lighting device that generates and diffuses light from below the object under measurement toward the object under measurement and the scanner. Distinguishment reliability and accuracy of a material of a used object under measurement can be improved.

## Claims

1. A system for distinguishing a material of an object under measurement, the system comprising:
a lighting device including a light emitting unit configured to emit light toward an object under measurement; and
a scanner including a near infrared ray (NIR) sensor and configured to distinguish a material of the object under measurement based on light generated by the NIR sensor and the light emitting unit and received after passing through the object under measurement and previously input information.

2. The system of claim 1, wherein the input information to the scanner is a wavelength band of light corresponding to the material of the object under measurement to be distinguished.

3. The system of claim 2, wherein the scanner is configured to:
distinguish a plastic material of the object under measurement; and
receive information on a wavelength band of light corresponding to at least one type of plastic material corresponding to the plastic material to be distinguished.

4. The system of claim 1, wherein the lighting device is disposed to face the scanner, and the object under measurement is positioned between the lighting device and the scanner.

5. The system of claim 1, wherein the lighting device has a seating portion on which the object under measurement is seated.

6. The system of claim 1, wherein the light emitting unit includes a halogen lamp.

7. The system of claim 6, wherein, in the lighting device, the light emitting unit is spaced a predetermined distance from the NIR sensor and disposed in a circumferential direction of the NIR sensor.

8. The system of claim 1, wherein the lighting device further includes a diffusion member configured to diffuse the light generated by the light emitting unit before the light reaches the object under measurement.

9. The system of claim 8, wherein the lighting device includes:
a controller connected to the light emitting unit and configured to control the light emitting unit to be turned ON/OFF;
a battery configured to supply electric power to the light emitting unit; and
a switch connected to the controller, connected to the battery, and configured to operate the light emitting unit.

10. The system of claim 9, wherein the lighting device further includes a casing on which the object under measurement is seated, which has an internal space in which the battery and the controller are mounted, and which has an opening hole of which at least a partial area is open so that the light generated by the light emitting unit faces the object under measurement, and
the diffusion member is mounted on the opening hole of the casing.

11. The system of claim 10, wherein the controller includes a charging identification member connected to the battery and configured to display a charging state of the battery, and
the charging identification member includes a light emitting diode (LED) lamp mounted between the light emitting units and configured to identify the charging state of the battery through the opening hole.

12. The system of claim 11, wherein the battery is provided to be charged/discharged, and
a charging terminal connected to the battery and configured to supply electric power to the battery is provided in the controller.

13. The system of claim 12, wherein the switch and the charging terminal are exposed at a side surface of the casing.

14. The system of claim 12, further comprising a battery housing in which the battery is accommodated,
wherein the battery housing is mounted inside the casing.

15. The system of claim 12, wherein the casing includes at least one gripping protrusion on a side surface thereof.

16. The system of claim 12, wherein the casing includes at least one slip preventing member on a bottom surface thereof.

17. The system of claim 12, wherein the casing includes:
an upper casing in which the opening hole is formed and at least one coupling protrusion is provided; and
a lower casing in which the battery and the controller are mounted and at least one coupling groove to which the coupling protrusion is coupled is provided,
a support rib configured to support the battery accommodated in the lower casing protrudes from an inner surface of the upper casing, and
the upper casing and the lower casing are separatable from each other by the coupling protrusion and the coupling groove.
